# EUROPÄISCHE PATENTSCHRIFT

(11) **EP 2 448 915 B1**
(45) Veröffentlichungstag und Bekanntmachung des Hinweises auf die Patenterteilung: **04.12.2013**
(21) Anmeldenummer: 10725608.3
(22) Anmeldetag: 09.06.2010
(51) Int. Cl.: C07C 231/02

(54) **KONTINUIERLICHES VERFAHREN ZUR HERSTELLUNG VON AMIDEN ALIPHATISCHER CARBONSÄUREN**
CONTINUOUS METHOD FOR PRODUCING AMIDES OF ALIPHATIC CARBOXYLIC ACIDS
PROCÉDÉ DE PRODUCTION CONTINUE D'AMIDES D'ACIDES CARBOXYLIQUES ALIPHATIQUES

(30) Priorität: 30.06.2009 DE 102009031057
(43) Veröffentlichungstag der Anmeldung: 09.05.2012
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2010/003445
(87) Internationale Veröffentlichungsnummer: WO 2011/000462

(56) Entgegenhaltungen:
- WO-A1-2008/043493
- US-A- 5 387 397
- US-A- 6 017 426
- PERREUX L ET AL: "Microwave effects in solvent-free esters aminolysis" TETRAHEDRON, ELSEVIER SCIENCE PUBLISHERS, AMSTERDAM, NL LNKD- DOI:10.1016/S0040-4020(03)00151-0, Bd. 59, Nr. 12, 17. März 2003 (2003-03-17) , Seiten 2185-2189, XP004414169 ISSN: 0040-4020

## Beschreibung

Die vorliegende Erfindung betrifft ein kontinuierliches Verfahren zur Herstellung von Amiden aliphatischer Carbonsäuren unter Mikrowellenbestrahlung im technischen Maßstab.

Amide finden als chemische Rohstoffe vielfältige Verwendung. Beispielsweise sind Amide niederer aliphatischer Carbonsäuren aprotische, polare Flüssigkeiten mit hervorragendem Lösevermögen. Fettsäureamide werden insbesondere auf Grund ihrer Oberflächenaktivität beispielsweise als Lösemittel, als Bestandteil von Wasch- und Reinigungsmitteln sowie in Kosmetika eingesetzt. Weiterhin werden sie erfolgreich als Hilfsmittel in der Metallbearbeitung, bei der Formulierung von Pflanzenschutzmitteln, als Antistatika für Polyolefine sowie bei der Förderung und Verarbeitung von Erdöl verwendet. Darüber hinaus sind Carbonsäureamide auch wichtige Rohstoffe zur Herstellung verschiedenster Pharmazeutika und Agrochemikalien.

Neben der Veresterung freier Carbonsäuren mit Aminen sind zur Herstellung von Carbonsäureamiden insbesondere im technischen Maßstab die Umsetzung reaktiver Carbonsäurederivate wie beispielsweise von Säurechloriden, -anhydriden sowie -estern mit den entsprechenden Aminen von Bedeutung. Während die Synthese von Amiden ausgehend von Säurechloriden zu mindestens equimolaren Mengen an zu entsorgenden Salzen und unerwünschten Restgehalten der Amide an Halogenidionen führt, ist die Reaktivität insbesondere der leicht zugänglichen Ester von Carbonsäuren mit aliphatischen Alkoholen gegenüber Aminen vergleichsweise niedrig, so dass diese Aminolyse lange Reaktionszeiten, hohe Temperaturen und/oder stark basische Katalysatoren erfordert. Unter diesen Reaktionsbedingungen treten oftmals unerwünschte Nebenreaktionen wie beispielsweise eine Oxidation des Amins, eine thermische Disproportionierung sekundärer Amine zu primärem und tertiärem Amin und/oder eine Decarboxylierung der Carbonsäure auf. Dadurch werden die Eigenschaften der Zielprodukte wie beispielsweise ihre Farbe beeinträchtigt, die Ausbeute herabgesetzt und oftmals werden zusätzliche Aufarbeitungsschritte notwendig.

Ein neuerer Ansatz zur Synthese von Amiden ist die durch Mikrowellen unterstützte Umsetzung von Carbonsäureestern mit Aminen zu Amiden. Diese lässt sich auch lösemittelfrei durchführen und bedingt damit neben erhöhten Raum-Zeit-Ausbeuten auch verringerte Umweltbelastungen.

Zradni et al. (Synth. Commun. 2002, 32, 3525-3531) offenbaren die Herstellung von Amiden durch Umsetzung von Estern verschiedener Carbonsäuren mit Aminen in Gegenwart größerer, d.h. bis zu equimolaren Mengen von Kalium-tert.-butanolat unter Mikrowelleneinfluss. Dabei wird im mmol-Maßstab gearbeitet.

Perreux et al. (Tetrahedron 59 (2003), 2185-2189) offenbaren die Herstellung von Carbonsäureamiden durch mikrowellenunterstützte Aminolyse von Carbonsäureestern mit primären Aminen. Dabei wird mit einem Monomode-Reaktor im Labormaßstab gearbeitet.

Das Scale-Up derartiger mikrowellenunterstützter Aminolysen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge und Plasmabildung enge Grenzen gesetzt. Weiterhin bereitet die in diesen Multimode-Mikrowellengeräten zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes Probleme bei der Maßstabsvergrößerung. Zudem bereitet dabei der sich während der Reaktion oftmals ändernde Mikrowellen-Absorptionskoeffizient des Reaktionsgemischs Schwierigkeiten hinsichtlich einer sicheren und reproduzierbaren Reaktionsführung.

WO 90/03840 offenbart ein kontinuierliches Verfahren zur Durchführung verschiedener chemischer Reaktionen in einem kontinuierlichen Labor-Mikrowellenreaktor. So wird beispielsweise Dimethylsuccinat mit Ammoniak bei 135 °C mit 51 %iger Ausbeute zum Succinamid umgesetzt. Die erzielten Ausbeuten wie auch das Reaktionsvolumen von 24 ml der im Multimode betriebenen Mikrowelle erlauben jedoch kein Up-Scaling in den großtechnischen Bereich. Der Wirkungsgrad dieses Verfahrens bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung kann zu unerwünschten Plasma- Entladungen oder zu so genannten thermischen Runaway-Effekten führen. Weiterhin machen die als Hot-Spots bezeichneten, sich zeitlich verändernden räumlichen Inhomogenitäten des Mikrowellenfeldes im Applikatorraum eine sichere und reproduzierbare Reaktionsführung im großen Maßstab unmöglich.

WO-08/043493 lehrt ein Verfahren zur Herstellung von Fettsäurealkanolamiden, indem mindestens ein Amin, das mindestens eine primäre oder sekundäre Aminogruppe und mindestens eine Hydroxylgruppe enthält, mit mindestens einer Fettsäure zu einem Ammoniumsalz umgesetzt wird, und dieses Ammoniumsalz nachfolgend unter Mikrowellenbestrahlung weiter zum Alkanolamid umgesetzt wird.

Weiterhin sind Monomode- bzw. Singlemode-Mikrowellenapplikatoren bekannt, bei denen mit einem einzigen Wellen-Modus gearbeitet wird, der sich in nur einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) auf kleine Reaktionsvolumina (≤ 50 ml) im Labormaßstab beschränkt.

Es wurde daher ein Verfahren zur Herstellung von Amiden aliphatischer Carbonsäuren gesucht, bei dem Carbonsäureester und Amin auch im technischen Maßstab unter Mikrowellenbestrahlung zum Amid umgesetzt werden können. Dabei sollen bei möglichst kurzen Reaktionszeiten möglichst hohe, das heißt bis zu quantitative Umsetzungsraten erzielt werden. Das Verfahren soll weiterhin eine möglichst energiesparende Herstellung der Carbonsäureamide ermöglichen, das heißt, die eingesetzte Mikrowellenleistung soll möglichst quantitativ vom Reaktionsgut absorbiert werden und das Verfahren somit einen hohen energetischen Wirkungsgrad bieten. Dabei sollen keine bzw. nur untergeordnete Mengen an Nebenprodukten anfallen. Die Amide sollen ferner eine geringe Eigenfärbung aufweisen. Zudem soll das Verfahren eine sichere und reproduzierbare Reaktionsführung gewährleisten.

Überraschenderweise wurde gefunden, dass sich Amide aliphatischer Carbonsäuren durch Umsetzung von Estern aliphatischer Carbonsäuren mit Aminen in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen herstellen lassen. Dabei wird die in den Mikrowellenapplikator eingestrahlte Mikrowellenenergie praktisch quantitativ vom Reaktionsgut absorbiert. Das erfindungsgemäße Verfahren besitzt zudem eine hohe Sicherheit bei der Durchführung und bietet eine hohe Reproduzierbarkeit der eingestellten Reaktionsbedingungen. Die nach dem erfindungsgemäßen Verfahren hergestellten Amide zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur Herstellung von Amiden aliphatischer Carbonsäuren, in dem mindestens ein Carbonsäureester der Formel (I)

R³-COOR⁴ (I)

worin
- R³: für Wasserstoff oder einen gegebenenfalls substituierten aliphatischen Kohlenwasserstoffrest mit 1 bis 100 Kohlenstoffatomen und
- R⁴: für einen gegebenenfalls substituierten Kohlenwasserstoffrest mit 1 bis 30 Kohlenstoffatomen steht,
oder worin R³ und R⁴ einen gegebenenfalls substituierten Ring mit 5, 6 oder 7 Ringgliedern bilden
mit mindestens einem Amin der Formel (II)

HNR¹R² (II)

worin R¹ und R² unabhängig voneinander für Wasserstoff oder einen gegebenenfalls substituierten Kohlenwasserstoffrest mit 1 bis 100 C-Atomen stehen,
und worin R¹ und R² unabhängig voneinander eine gegebenenfalls substituierte heteroaromatische Gruppe mit 5 bis 12 Ringgliedern sein können,
und worin R¹ und R² unabhängig voneinander für einen mit Heteroatomen unterbrochenen Alkylrest stehen können,
und worin R¹ und R² zusammen mit dem Stickstoffatom, an das sie gebunden sind, einen Ring bilden können,
unter Mikrowellenbestrahlung des Reaktionsgemischs in einem weitgehend mikrowellentransparenten Reaktionsrohr innerhalb eines mit einem Mikrowellengenerator über Wellenleiter verbundenen Hohlleiters, dessen Längsachse sich in der Ausbreitungsrichtung der Mikrowellen eines Monomode-Mikrowellenapplikators befindet, zum Carbonsäureamid umgesetzt wird.

Erfindungsgemäß bevorzugte Ester der Formel (I) leiten sich von Carbonsäuren der Formel (III)

R³COOH (III)

und Alkoholen der Formel (IV)

R⁴OH (IV)

ab, wobei R³ und R⁴ die oben angegebenen Bedeutungen haben,
aus denen sie sich nach bekannten Methoden wie beispielsweise durch Kondensation herstellen lassen.

Unter aliphatischen Carbonsäuren III werden hier allgemein Verbindungen verstanden, die mindestens eine Carboxylgruppe an einem gegebenenfalls substituierten aliphatischen Kohlenwasserstoffrest mit 1 bis 100 C-Atomen besitzen sowie Ameisensäure. In einer bevorzugten Ausführungsform ist der aliphatische Kohlenwasserstoffrest R³ ein unsubstituierter Alkyl- oder Alkenylrest. In einer weiteren bevorzugten Ausführungsform trägt der aliphatische Kohlenwasserstoffrest einen oder mehrere wie beispielsweise zwei, drei, vier oder mehr weitere Substituenten. Geeignete Substituenten sind beispielsweise Hydroxy-, C₁-C₅-Alkoxy- wie beispielsweise Methoxy-, Poly(C₁-C₅-Alkoxy)-, Poly(C₁-C₅-Alkoxy)alkyl-, Ester-, Keto-, Amid-, Cyano-, Nitril-, Nitro- und/oder Arylgruppen mit 5 bis 20 Kohlenstoffatomen wie beispielsweise Phenylgruppen, mit der Maßgabe, dass diese Substituenten unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten wie beispielsweise C₁-C₂₀-Alkyl-, C₂-C₂₀-Alkenyl-, C₁-C₅-Alkoxy- wie beispielsweise Methoxy-, Ester-, Amid-, Cyano-, Nitril-, und/oder Nitrogruppen tragen. Der aliphatische Kohlenwasserstoffrest trägt jedoch höchstens so viele Substituenten wie er Valenzen hat.

Der Kohlenwasserstoffrest des aliphatischen Carbonsäureesters trägt vorzugsweise als weitere Substituenten keine freien Carbonsäure- bzw. Carboxylatgruppen; diese könnten ihrerseits mit den Aminen der Formel (II) zu unerwünschten Nebenprodukten reagieren.

In einer speziellen Ausführungsform trägt der aliphatische Kohlenwasserstoffrest R³ mindestens eine weitere Estergruppe -COOR⁴. So ist das erfindungsgemäße Verfahren ebenso zur Umsetzung von Polycarbonsäureestern, die sich von Polycarbonsäuren mit zwei oder mehr wie beispielsweise zwei, drei, vier oder mehr Carboxylgruppen ableiten zu Polycarbonsäureamiden geeignet. Die Estergruppen können gemäß dem erfindungsgemäßen Verfahren vollständig oder auch nur teilsweise in Amide überführt werden. Der Amidierungsgrad lässt sich beispielsweise durch die Stöchiometrie zwischen Carbonsäureester und Amin im Reaktionsgemisch einstellen.

Erfindungsgemäß besonders bevorzugt sind aliphatische Carbonsäureester der Formel (I), die sich von Carbonsäuren mit einem gegebenenfalls substituierten aliphatischen Kohlenwasserstoffrest R³ mit 1 bis 50 C-Atomen und insbesondere mit 2 bis 26 C-Atomen wie beispielsweise mit 3 bis 20 C-Atomen ableiten. Sie können natürlichen oder synthetischen Ursprungs sein. Der aliphatische Kohlenwasserstoffrest kann auch Heteroatome wie beispielsweise Sauerstoff, Stickstoff, Phosphor und/oder Schwefel enthalten, bevorzugt jedoch nicht mehr als ein Heteroatom pro 3 C-Atome.

Die aliphatischen Kohlenwasserstoffreste können linear, verzweigt oder zyklisch sein. Die Estergruppe kann an einem primären, sekundären oder tertiären C-Atom gebunden sein. Bevorzugt ist sie an einem primären C-Atom gebunden. Die Kohlenwasserstoffreste können gesättigt oder, sofern ihr Kohlenwasserstoffrest R³ mindestens 2 Kohlenstoffatome umfasst, auch ungesättigt sein. Bevorzugte ungesättigte Kohlenwasserstoffreste, die keine zur Estergruppe konjugierte C=C-Doppelbindung tragen, enthalten bevorzugt eine oder mehrere C=C-Doppelbindungen und besonders bevorzugt eine, zwei oder drei C=C-Doppelbindungen. So hat sich das erfindungsgemäße Verfahren besonders zur Herstellung von Amiden mehrfach ungesättigter Carbonsäuren bewährt, da die Doppelbindungen der ungesättigten Carbonsäuren unter den Reaktionsbedingungen des erfindungsgemäßen Verfahrens nicht angegriffen werden. Bevorzugte zyklische aliphatische Kohlenwasserstoffreste besitzen mindestens einen Ring mit vier, fünf, sechs, sieben, acht oder mehr Ringatomen.

In einer bevorzugten Ausführungsform steht R³ für einen gesättigten Alkylrest mit 1, 2, 3 oder 4 C-Atomen. Dieser kann linear oder bei mindestens 3 C-Atomen auch verzweigt sein. Die Estergruppe kann an einem primären, sekundären oder, wie im Falle der Pivalinsäure, tertiären C-Atom gebunden sein. In einer besonders bevorzugten Ausführungsform ist der Alkylrest ein unsubstituierter Alkylrest. In einer weiteren besonders bevorzugten Ausführungsform trägt der Alkylrest einen bis neun, bevorzugt einen bis fünf wie beispielsweise zwei, drei oder vier weitere der oben genannten Substituenten. Der Alkylrest trägt jedoch höchstens so viele Substituenten, wie er Valenzen hat. Bevorzugte weitere Substituenten sind Estergruppen sowie gegebenenfalls substituierte C₅-C₂₀-Arylreste.

In einer weiteren bevorzugten Ausführungsform leiten sich die Carbonsäureester der Formel (I) von ethylenisch ungesättigten Carbonsäuren ab. Dabei steht R³ für eine gegebenenfalls substituierte Alkenylgruppe mit 2 bis 4 Kohlenstoffatomen. Unter ethylenisch ungesättigten Carbonsäuren werden hier solche Carbonsäuren verstanden, die eine zur Carboxylgruppe konjugierte C=C-Doppelbindung besitzen. Die Alkenylgruppe kann linear oder, sofern sie mindestens drei C-Atome umfasst, verzweigt sein. In einer bevorzugten Ausführungsform ist der Alkenylrest ein unsubstituierter Alkenylrest. Besonders bevorzugt ist R³ ein Alkenylrest mit 2 oder 3 C-Atomen. In einer weiteren bevorzugten Ausführungsform trägt der Alkenylrest einen oder mehrere, wie beispielsweise zwei, drei oder mehr weitere der oben genannten Substituenten. Der Alkenylrest trägt jedoch höchstens so viele Substituenten, wie er Valenzen hat. In einer bevorzugten Ausführungsform trägt der Alkenylrest R³ ethylenisch ungesättigter Carbonsäuren als weiteren Substituenten eine Estergruppe oder eine gegebenenfalls substituierte C₅-C₂₀-Arylgruppe. So ist das erfindungsgemäße Verfahren ebenso zur Umsetzung von ethylenisch ungesättigten Dicarbonsäureestern geeignet.

In einer weiteren bevorzugten Ausführungsform leiten sich die Carbonsäureester (I) von Fettsäuren ab. Dabei steht R³ für einen gegebenenfalls substituierten aliphatischen Kohlenwasserstoffrest mit 5 bis 50 C-Atomen. Besonders bevorzugt leiten sie sich dabei von Fettsäuren ab, die einen aliphatischen Kohlenwasserstoffrest mit 6 bis 30 C-Atomen und insbesondere mit 7 bis 26 C-Atomen wie beispielsweise mit 8 bis 22 C-Atomen tragen. In einer bevorzugten Ausführungsform ist der Kohlenwasserstoffrest der Fettsäure ein unsubstituierter Alkyl- oder Alkenylrest. In einer weiteren bevorzugten Ausführungsform trägt der Kohlenwasserstoffrest der Fettsäure einen oder mehrere wie beispielsweise zwei, drei, vier oder mehr weitere Substituenten. In einer speziellen Ausführungsform trägt der Kohlenwasserstoffrest der Fettsäure eine, zwei, drei, vier oder mehr weitere Carbonsäureestergruppen.

Zur Amidierung gemäß dem erfindungsgemäßen Verfahren geeignete Carbonsäureester (I) leiten sich beispielsweise von Ameisensäure, Essigsäure, Propionsäure, Milchsäure, Buttersäure, iso-Buttersäure, Pentansäure, iso-Pentansäure, Pivalinsäure, Acrylsäure, Methacrylsäure, Crotonsäure, 2,2-Dimethylacrylsäure, Maleinsäure, Fumarsäure, Itaconsäure, Zimtsäure und Methoxyzimtsäure, Malonsäure, Bernsteinsäure, Butantetracarbonsäure, Phenylessigsäure, (Methoxyphenyl)essigsäure, (Dimethoxyphenyl)essigsäure, 2-Phenylpropionsäure, 3-Phenylpropionsäure, 3-(4-Hydroxyphenyl)propionsäure, 4-Hydroxyphenoxyessigsäure, Acetessigsäure, Hexansäure, Cyclohexansäure, Heptansäure, Octansäure, Nonansäure, Neononansäure, Decansäure, Neodecansäure, Undecansäure, Neoundecansäure, Dodecansäure, Tridecansäure, iso-Tridecansäure, Tetradecansäure, 12-Methyltridecansäure, Pentadecansäure, 13-Methyltetradecansäure, 12-Methyltetradecansäure, Hexadecansäure, 14-Methylpentadecansäure, Heptadecansäure, 15-Methylhexadecansäure, 14-Methylhexadecansäure, Octadecansäure, Iso-Octadecansäure, Icosansäure, Docosansäure und Tetracosansäure, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure, Dodecenylbernsteinsäure und Octadecenylbemsteinsäure und aus ungesättigten Fettsäuren herstellbare Dimerfettsäuren sowie deren Mischungen ab. Weiterhin geeignet sind aus natürlichen Fetten und Ölen wie beispielsweise Baumwollsamen-, Cocos-, Erdnuss-, Färberdistel-, Mais-, Palmkern-, Raps-, Oliven-, Senfsamen, Soja-, Sonnenblumenöl sowie Talg-, Knochen- und Fischöl gewonnene Carbonsäureestermischungen. Als Carbonsäureester bzw. Carbonsäureestermischungen für das erfindungsgemäße Verfahren ebenfalls geeignete Ester leiten sich von Tallölfettsäure sowie Harz- und Naphthensäuren ab.

In einer bevorzugten Ausführungsform steht R⁴ für einen aliphatischen Rest. Dieser hat bevorzugt 1 bis 24, besonders bevorzugt 2 bis 18 und speziell 3 bis 6 C-Atome. Der aliphatische Rest kann linear oder, sofern er mindestens 3 C-Atome umfasst, verzweigt oder zyklisch sein. Er kann weiterhin gesättigt oder, sofern er mindestens 3 C-Atome besitzt, ungesättigt sein, bevorzugt ist er gesättigt. Gegebenenfalls kann der Kohlenwasserstoffrest R⁴ Substituenten wie beispielsweise C₅-C₂₀-Arylgmppen tragen und/oder mit Herteroatomen wie beispielsweise Sauerstoff und/oder Stickstoff unterbrochen sein. Besonders bevorzugte aliphatische Reste R⁴ sind Methyl, Ethyl, n-Propyl, iso-Propyl, n-Butyl, iso-Butyl und tert.-Butyl, n-Hexyl, Cyclohexyl, n-Octyl, n-Decyl, n-Dodecyl, Tridecyl, Isotridecyl, Tetradecyl, Hexadecyl, Octadecyl und Methylphenyl.

In einer speziellen Ausführungsform leiten sich die Ester der Formel (I) von Alkoholen der Formel (IV) ab, deren aliphatischer Rest R⁴ eine oder mehrere wie beispielsweise zwei, drei, vier, fünf, sechs oder mehr weitere Hydroxylgruppen trägt. Die Hydroxylgruppen können an benachbarten C-Atomen oder auch an weiter entfernten Kohlenstoffatomen des Kohlenwasserstoffrestes gebunden sein, jedoch höchstens eine OH-Gruppe pro Kohlenstoffatom. Die OH-Gruppen der den Estern (I) zu Grunde liegenden Polyole können dabei vollständig oder auch nur teilweise verestert sein. Sie können mit gleichen oder verschiedenen Carbonsäuren verestert sein. So eignet sich das erfindungsgemäße Verfahren insbesondere zur Umsetzung von Estern, die sich von Polyolen wie beispielsweise Ethylenglykol, 1,2-Propandiol, 1,3-Propandiol, Neopentylglykol, Glycerin, Sorbit, Pentaerythrit, Fructose und Glucose ableiten. In einer bevorzugten Ausführungsform werden Triglyceride und insbesondere Triglyceride biogener Herkunft eingesetzt. Der Amidierungsgrad lässt sich dabei beispielsweise über die Stöchiometrie zwischen Carbonsäureestergruppen und Aminogruppen im Reaktionsgemisch steuern.

In einer weiteren bevorzugten Ausführungsform steht R⁴ für eine gegebenenfalls substituierte C₆-C₁₂-Arylgruppe oder eine gegebenenfalls substituierte heteroaromatische Gruppe mit 5 bis 12 Ringgliedern. Bevorzugte Heteroatome sind Sauerstoff, Stickstoff und Schwefel. Bevorzugte Substituenten sind beispielsweise Nitrogruppen. Ein besonders bevorzugter aromatischer Rest R⁴ ist der Nitrophenylrest.

Beispiele für geeignete Alkohole (IV), von denen sich die Ester der Formel (I) ableiten sind Methanol, Ethanol, n-Propanol, iso-Propanol, n-Butanol, iso-Butanol, tert.-Butanol, Pentanol, Neopentanol, n-Hexanol, iso-Hexanol, Cyclohexanol, Heptanol, Octanol, Decanol, Dodecanol, Tetradecanol, Hexadecanol, Octadecanol, Eicosanol, Ethylenglykol, 2-Methoxyethanol, Propylenglykol, Glycerin, Sorbitan, Sorbitol, Diethylenglykol, Triethylenglykol, Polyethylenglykol, Polypropylenglykol, Triethanolamin, N,N-Dimethylethanolamin, N,N-Diethylethanolamin, Phenol, Naphthol und deren Mischungen. Weiterhin geeignet sind aus natürlichen Rohstoffen gewonnene Fettalkoholmischungen wie beispielsweise Cocosfettalkohol, Palmkernfettalkohol und Talgfettalkohol. Besonders bevorzugt sind niedere aliphatische Alkohole wie Methanol, Ethanol, Propanol, n-Butanol sowie Glycerin.

Beispiele für erfindungsgemäß besonders geeignete Ester der Formel (I) sind Ester aus aliphatischen Carbonsäuren und Monoalkoholen mit 1 bis 4 C-Atomen wie beispielsweise Fettsäuremethylester und Triglyceride von Fettsäuren wie beispielsweise Triolein, Tristearin und biogene Öle und Fette.

Auch intra- sowie intermolekulare Ester von Hydroxycarbonsäuren können nach dem erfindungsgemäßen Verfahren in Amide überführt werden, wobei die Hydroxylgruppe bei der Aminolyse insbesondere mit höchstens equimolaren Mengen (bezogen auf die Estergruppen) an Amin der Formel (II) erhalten bleibt. Beispiele für derartige Ester sind Lactone wie Caprolacton und Lactid (3,6-Dimethyl-1,4-dioxan-2,5-dion).

Das erfindungsgemäße Verfahren eignet sich bevorzugt zur Herstellung sekundärer Amide. Dazu werden Carbonsäureester (I) mit Aminen (II), bei denen R¹ für einen Kohlenwasserstoffrest mit 1 bis 100 Kohlenstoffatomen und R² für Wasserstoff steht, umgesetzt.

Das erfindungsgemäße Verfahren eignet sich weiterhin besonders bevorzugt zur Herstellung tertiärer Amide. Dazu werden Carbonsäureester (I) mit Aminen (II), bei denen beide Reste R¹ und R² unabhängig voneinander für einen Kohlenwasserstoffrest mit 1 bis 100 Kohlenstoffatomen stehen, umgesetzt. Die Reste R¹ und R² können dabei gleich oder verschieden sein. In einer besonders bevorzugten Ausführungsform sind R¹ und R² gleich.

In einer ersten bevorzugten Ausführungsform stehen R¹ und/oder R² unabhängig voneinander für einen aliphatischen Rest. Dieser hat bevorzugt 1 bis 24, besonders bevorzugt 2 bis 18 und speziell 3 bis 6 C-Atome. Der aliphatische Rest kann linear, verzweigt oder zyklisch sein. Er kann weiterhin gesättigt oder ungesättigt sein. Der Kohlenwasserstoffrest kann Substituenten tragen. Solche Substituenten können beispielsweise Hydroxy-, C₁-C₅-Alkoxy-, Alkoxyalkyl-, Cyano-, Nitril-, Nitro- und/oder C₅-C₂₀-Arylgruppen wie beispielsweise Phenylreste sein. Die C₅-C₂₀-Arylgruppen können ihrerseits gegebenenfalls mit Halogenatomen, C₁-C₂₀-Alkyl-, C₂-C₂₀-Alkenyl-, Hydroxyl-, C₁-C₅-Alkoxy- wie beispielsweise Methoxy-, Ester-, Amid-, Cyano-, Nitril-, und/oder Nitrogruppen substituiert sein. Besonders bevorzugte aliphatische Reste sind Methyl, Ethyl, Hydroxyethyl, n-Propyl, iso-Propyl, Hydroxypropyl, n-Butyl, iso-Butyl und tert.-Butyl, Hydroxybutyl, n-Hexyl, Cyclohexyl, n-Octyl, n-Decyl, n-Dodecyl, Tridecyl, Isotridecyl, Tetradecyl, Hexadecyl, Octadecyl und Methylphenyl. In einer besonders bevorzugten Ausführungsform stehen R¹ und/oder R² unabhängig voneinander für Wasserstoff, einen C₁-C₆-Alkyl-, C₂-C₆-Alkenyl- oder C₃-C₆-Cycloalkylrest und speziell für einen Alkylrest mit 1, 2, oder 3 C-Atomen. Diese Reste können bis zu drei Substituenten tragen.

In einer weiteren bevorzugten Ausführungsform bilden R¹ und R² zusammen mit dem Stickstoffatom, an das sie gebunden sind, einen Ring. Dieser Ring hat bevorzugt 4 oder mehr wie beispielsweise mit 4, 5, 6 oder mehr Ringglieder. Bevorzugte weitere Ringglieder sind dabei Kohlenstoff-, Stickstoff-, Sauerstoff- und Schwefelatome. Die Ringe können ihrerseits wiederum Substituenten wie beispielsweise Alkylreste tragen. Geeignete Ringstrukturen sind beispielsweise Morpholinyl-, Pyrrolidinyl, Piperidinyl-, Imidazolyl- und Azepanylreste.

In einer weiteren bevorzugten Ausführungsform stehen R¹ und/oder R² unabhängig voneinander für eine gegebenenfalls substituierte C₆-C₁₂-Arylgruppe oder eine gegebenenfalls substituierte heteroaromatische Gruppe mit 5 bis 12 Ringgliedern. Bevorzugte Heteroatome heteroaromatischer Gruppen sind Sauerstoff, Stickstoff und/oder Schwefel.

In einer weiteren bevorzugten Ausführungsform stehen R¹ und/oder R² unabhängig voneinander für einen mit Heteroatomen unterbrochenen Alkylrest. Besonders bevorzugte Heteroatome sind Sauerstoff und Stickstoff.

So stehen R¹ und/oder R² unabhängig voneinander bevorzugt für Reste der Formel (V)

-(R⁷O)ₙ-R⁸ (V)

worin
- R⁷: für eine Alkylengruppe mit 2 bis 6 C-Atomen und bevorzugt mit 2 bis 4 C-Atomen wie beispielsweise Ethylen, Propylen, Butylen oder Mischungen daraus,
- R⁸: für Wasserstoff, einen Kohlenwasserstoffrest mit 1 bis 24 C-Atomen oder eine Gruppe der Formel -R⁷-NR¹¹R¹²,
- n: für eine Zahl zwischen 2 und 50, bevorzugt zwischen 3 und 25 und insbesondere zwischen 4 und 10 und
- R¹¹, R¹²: unabhängig voneinander für einen aliphatischen Rest mit 1 bis 24 C-Atomen und bevorzugt 2 bis 18 C-Atomen, eine Aryl- oder Heteroarylgruppe mit 5 bis 12 Ringgliedern, eine Poly(oxyalkylen)gruppe mit 1 bis 50 Poly(oxyalkylen)einheiten, wobei sich die Poly(oxyalkylen)einheiten von Alkylenoxideinheiten mit 2 bis 6 C-Atomen ableiten, oder R¹¹ und R¹² gemeinsam mit dem Stickstoffatom, an das sie gebunden sind, einen Ring mit 4, 5, 6 oder mehr Ringgliedern bilden, stehen.

Weiterhin bevorzugt stehen R¹ und/oder R² unabhängig voneinander für Reste der Formel (VI)

-[R⁹-N(R¹⁰)]ₘ-(R¹⁰) (VI)

worin
- R⁹: für eine Alkylengruppe mit 2 bis 6 C-Atomen und bevorzugt mit 2 bis 4 C-Atomen wie beispielsweise Ethylen, Propylen oder Mischungen daraus steht,
- jedes R¹⁰: unabhängig voneinander für Wasserstoff, einen Alkyl- oder Hydroxyalkylrest mit bis zu 24 C-Atomen wie beispielsweise 2 bis 20 C-Atomen, einen Polyoxyalkylenrest -(R⁷-O)ₚ-R⁸, oder einen Polyiminoalkylenrest -[R⁹-N(R¹⁰)]_{q}-(R¹⁰) stehen, wobei R⁷, R⁸, R⁹ und R¹⁰ die oben gegebenen Bedeutungen haben und q und p unabhängig voneinander für 1 bis 50 stehen und
- m: für eine Zahl von 1 bis 20 und bevorzugt 2 bis 10 wie beispielsweise drei, vier, fünf oder sechs steht. Die Reste der Formel IV enthalten vorzugsweise 1 bis 50, insbesondere 2 bis 20 Stickstoffatome.

In einer speziellen Ausführungsform eignet sich das erfindungsgemäße Verfahren zur Herstellung tertiäre Aminogruppen tragender und damit basischer Carbonsäureamide, wobei mindestens ein aliphatischer Carbonsäureester (I) mit mindestens einem eine primäre und/oder sekundäre und mindestens eine tertiäre Aminogruppe tragenden Polyamin unter Mikrowellenbestrahlung in einem Reaktionsrohr, dessen Längsachse sich in der Ausbreitungsrichtung der Mikrowellen eines Monomode-Mikrowellenapplikators befindet, zum basischen Carbonsäureamid umgesetzt wird. Unter tertiären Aminogruppen werden hierbei Struktureinheiten verstanden, in denen ein Stickstoffatom kein acides Proton trägt. So kann der Stickstoff der tertiären Aminogruppe beispielsweise drei Kohlenwasserstoffreste tragen oder auch Bestandteil eines heterozyklischen Systems sein. Bei dieser Ausführungsform steht R¹ bevorzugt für eine der oben angegebenen Bedeutungen, besonders bevorzugt für Wasserstoff, einen aliphatischen Rest mit 1 bis 24 C-Atomen oder eine Arylgruppe mit 6 bis 12 C-Atomen und speziell für Methyl, und R² für einen tertiäre Aminogruppen tragenden Kohlenwasserstoffrest der Formel (VII)

-(A)ₛ-Z (VII)

worin
- A: für einen Alkylenrest mit 1 bis 12 C-Atomen, einen Cycloalkylenrest mit 5 bis 12 Ringgliedern, einen Arylenrest mit 6 bis 12 Ringgliedem oder einen Heteroarylenrest mit 5 bis 12 Ringgliedern
- s: für 0 oder 1,
- Z: für eine Gruppe der Formel -NR¹³R¹⁴ oder für einen Stickstoff enthaltenden zyklischen Kohlenwasserstoffrest mit mindestens 5 Ringgliedern und
- R¹³ und R¹⁴: unabhängig voneinander für C₁- bis C₂₀-Kohlenwasserstoffreste oder für Polyoxyalkylenreste der Formel -(R⁷-O)ₚ-R⁸ (III) stehen, wobei R⁷, R⁸ und p die oben gegebenen Bedeutungen haben.

Bevorzugt steht A für einen Alkylenrest mit 2 bis 24 C-Atomen, einen Cycloalkylenrest mit 5 bis 12 Ringgliedern, einen Arylenrest mit 6 bis 12 Ringgliedern oder einen Heteroarylenrest mit 5 bis 12 Ringgliedern. Besonders bevorzugt steht A für einen Alkylenrest mit 2 bis 12 C-Atomen. Bevorzugt steht s für 1. Besonders bevorzugt stehen A für einen linearen oder verzweigten Alkylenrest mit 1 bis 6 C-Atomen und s für 1.

Weiterhin bevorzugt steht A, wenn Z für eine Gruppe der Formel -NR¹³R¹⁴ steht, für einen linearen oder verzweigten Alkylenrest mit 2, 3 oder 4 C-Atomen, insbesondere für einen Ethylenrest oder einen linearen Propylenrest. Steht Z dagegen für einen Stickstoff enthaltenden zyklischen Kohlenwasserstoffrest, so sind Verbindungen besonders bevorzugt, in denen A für einen linearen Alkylenrest mit 1, 2 oder 3 C-Atomen, insbesondere für einen Methylen-, Ethylen- oder einen linearen Propylenrest steht.

Für das Strukturelement A bevorzugte zyklische Reste können mono- oder polyzyklisch sein und beispielsweise zwei oder drei Ringsysteme enthalten. Bevorzugte Ringsysteme besitzen 5, 6 oder 7 Ringglieder. Bevorzugt enthalten sie insgesamt etwa 5 bis 20 C-Atome, insbesondere 6 bis 10 C-Atome. Bevorzugte Ringsysteme sind aromatisch und enthalten nur C-Atome. In einer speziellen Ausführungsform werden die Strukturelemente A aus Arylenresten gebildet. Das Strukturelement A kann Substituenten wie beispielsweise Alkylreste, Nitro-, Cyano-, Nitril-, Oxyacyl- und/oder Hydroxyalkylgruppen tragen. Ist A ein monozyklischer aromatischer Kohlenwasserstoff, so befinden sich die Aminogruppen bzw. Aminogruppen tragenden Substituenten bevorzugt in ortho- oder para-Stellung zueinander.

Bevorzugt steht Z für eine Gruppe der Formel -NR¹³R¹⁴. Darin stehen R¹³ und R¹⁴ unabhängig voneinander bevorzugt für aliphatische, aromatische und/oder araliphatische Kohlenwasserstoffreste mit 1 bis 20 Kohlenstoffatomen. Als R¹³ und R¹⁴ besonders bevorzugt sind Alkylreste. Sind R¹³ und/oder R¹⁴ Alkylreste, so tragen sie bevorzugt 1 bis 14 C-Atome wie beispielsweise 1 bis 6 C-Atome. Diese Alkylreste können linear, verzweigt und/oder zyklisch sein. Besonders bevorzugt stehen R¹³ und R¹⁴ für Alkylreste mit 1 bis 4 C-Atomen wie beispielsweise Methyl, Ethyl, n-Propyl, iso-Propyl, n-Butyl und iso-Butyl. In einer weiteren Ausführungsform stehen die Reste R¹³ und/oder R¹⁴ unabhängig voneinander für Polyoxyalkylenreste der Formel (III).

Als R¹³ und/oder R¹⁴ besonders geeignete aromatische Reste umfassen Ringsysteme mit mindestens 5 Ringgliedern. Sie können Heteroatome wie S, O und N enthalten. Als R¹³ und/oder R¹⁴ besonders geeignete araliphatische Reste umfassen Ringsysteme mit mindestens 5 Ringgliedern, die über einen C₁-C₆-Alkylrest an den Stickstoff gebunden sind. Sie können Heteroatome wie S, O und N enthalten. Die aromatischen wie auch die araliphatischen Reste können weitere Substituenten wie beispielsweise Alkylreste, Nitro-, Cyano-, Nitril-, Oxyacyl- und/oder Hydroxyalkylgruppen tragen.

In einer weiteren bevorzugten Ausführungsform steht Z für einen Stickstoff enthaltenden, zyklischen Kohlenwasserstoffrest, dessen Stickstoffatom nicht zur Bildung von Amiden befähigt ist. Das zyklische System kann mono-, di- oder auch polyzyklisch sein. Bevorzugt enthält es einen oder mehrere fünf- und/oder sechsgliedrige Ringe. Dieser zyklische Kohlenwasserstoff kann ein oder mehrere wie beispielsweise zwei oder drei Stickstoffatome enthalten, die keine aciden Protonen tragen, besonders bevorzugt enthält er ein N-Atom. Besonders geeignet sind dabei stickstoffhaltige Aromaten, deren Stickstoff an der Ausbildung eines aromatischen π-Elektronensextetts beteiligt ist wie beispielsweise Pyridin. Gleichfalls geeignet sind stickstoffhaltige Heteroaliphaten, deren Stickstoffatome keine Protonen tragen und zum Beispiel sämtlich mit Alkylresten abgesättigt sind. Die Verknüpfung von Z mit A bzw. dem Stickstoff der Formel (II) erfolgt hier bevorzugt über ein Stickstoffatom des Heterozyklus wie beispielsweise bei 1-(3-Aminopropyl)pyrrolidin. Der durch Z repräsentierte zyklische Kohlenwasserstoff kann weitere Substituenten wie beispielsweise C₁-C₂₀-Alkylreste, Halogenatome, halogenierte Alkylreste, Nitro-, Cyano-, Nitril-, Hydroxyl- und/oder Hydroxyalkylgruppen tragen.

Je nach stöchiometrischem Verhältnis zwischen Carbonsäureester (I) und Polyamin (VI) werden ein oder mehrere Aminogruppen, die jeweils mindestens ein Wasserstoffatom tragen, in das Carbonsäureamid überführt. Bei der Umsetzung von Polycarbonsäureestern mit Polyaminen der Formel (VI) können insbesondere die primären Aminogruppen auch in Imide überführt werden.

Zur erfindungsgemäßen Herstellung von primären Amiden werden anstelle von Ammoniak bevorzugt stickstoffhaltige Verbindungen, die beim Erhitzen Ammoniakgas abspalten, eingesetzt. Beispiele für derartige stickstoffhaltige Verbindungen sind Harnstoff und Formamid.

Beispiele für geeignete Amine sind Ammoniak, Methylamin, Ethylamin, Propylamin, Butylamin, Hexylamin, Cyclohexylamin, Octylamin, Decylamin, Dodecylamin, Tetradecylamin, Hexadecylamin, Octadecylamin Dimethylamin, Diethylamin, Ethylmethylamin, Di-n-propylamin, Di-iso-propylamin, Dicyclohexylamin, Didecylamin, Didodecylamin, Ditetradecylamin, Dihexadecylamin, Dioctadedcylamin, Benzylamin, Phenylethylamin, Ethylendiamin, Diethylentriamin, Triethylentetramin, Tetraethylenpentamin sowie deren Mischungen. Beispiele für geeignete tertiäre Amingruppen tragende Amine sind N,N-Dimethylethylendiamin, N,N-Dimethyl-1,3-propandiamin, N,N-Diethyl-1,3-propandiamin, N,N-Dimethyl-2-methyl-1,3-propandiamin, 1-(3-Aminopropyl)pyrrolidin, 1-(3-Aminopropyl)-4-methylpiperazin, 3-(4-Morpholino)-1-propylamin, 2-Aminothiazol, die verschiedenen Isomere des N,N-Dimethylaminoanilins, des Aminopyridins, des Aminomethylpyridins, des Aminomethylpiperidins und des Aminochinolins, sowie 2-Aminopyrimidin, 3-Aminopyrazol, Aminopyrazin und 3-Amino-1,2,4-triazol. Auch Mischungen verschiedener Amine sind geeignet. Besonders bevorzugt sind davon Dimethylamin, Diethylamin, Di-n-propylamin, Di-iso-propylamin, Ethylmethylamin und N,N-Dimethylaminopropylamin.

Besonders bevorzugt ist das erfindungsgemäße Verfahren zur Herstellung von Amiden aliphatischer C₁-C₄-Alkylamine durch Umsetzung von Estern aus aliphatischen C₁-C₂₀-Carbonsäuren und aliphatischen C₁-C₆-Alkoholen mit primären oder sekundären aliphatischen C₁-C₄-Alkylaminen. Weiterhin besonders bevorzugt ist das erfindungsgemäße Verfahren zur Herstellung von basischen Amiden durch Umsetzung von Estern aus Fettsäuren und niederen aliphatischen C₁-C₄-Alkoholen mit Polyaminen, die mindestens eine primäre und/oder sekundäre sowie mindestens eine tertiäre Aminogrupppe tragen. Weiterhin besonders bevorzugt ist das erfindungsgemäße Verfahren zur Herstellung von Amiden durch Umsetzung von Estern aus Fettsäuren und Polyolen wie beispielsweise Triglyceriden biogenen Ursprungs mit primären oder sekundären aliphatischen Aminen mit C₁-C₄-Alkylresten. Weiterhin besonders bevorzugt ist das erfindungsgemäße Verfahren zur Herstellung von basischen Amiden durch Umsetzung von Estern aus Fettsäuren und Polyolen mit Polyaminen, die mindestens eine primäre und/oder sekundäre sowie mindestens eine tertiäre Aminogrupppe tragen.

Für den Fall, dass der Carbonsäureester (I) zwei oder mehr Estergruppen und das Amin (II) zwei oder mehr Aminogruppen enthält bzw. beide Reaktanden jeweils eine Ester- und eine Aminogruppe tragen, können gemäß dem erfindungsgemäßen Verfahren auch Polymere hergestellt werden. Dabei ist die während der Mikrowellenbestrahlung ansteigende Viskosität des Reaktionsgemischs bei der Auslegung der Apparatur zu beachten.

Das Verfahren ist insbesondere geeignet zur Herstellung von N,N-Dimethylformamid, N-Methylacetamid, N,N-Dimethylacetamid, N,N-Dipropylacetamid, N,N-Dimethylpropionamid, N,N-Dimethylbutyramid, N,N-Dimethyl(phenyl)essigsäureamid, N,N-Dimethyl(p-methoxyphenyl)essigsäureamid, N,N-Diethyl-2-phenylpropionsäure, N-Methyloctansäureamid, N,N-Dimethyloctansäureamid, N-Methylcocosfettsäureamid, N,N-Dimethylcocosfettsäureamid, N-Ethylcocosfettsäureamid, N,N-Diethylcocosfettsäureamid, N-Methylstearinsäureamid, N,N-Diethylstearinsäureamid, N,N-Dimethyltallölfettsäureamid, N-Stearylstearinsäureamid, N-(N',N'-Dimethylamino)proyl-cocosfettsäureamid, N-(N',N'-Dimethylamino)proyl-talgfettsäureamid, N-Ethylmandelsäureamid, N,N-Dimethylmilchsäureamid, Octansäurediethanolamid, Laurinsäuremonoethanolamid, Laurinsäurediethanolamid, Laurinsäurediglykolamid, Cocosfettsäurediglykolamid, Stearinsäuremonoethanolamid, Stearinsäurediethanolamid, Tallölfettsäuremonoethanolamid, Tallölfettsäurediethanolamid und Tallölfettsäurediglykolamid sowie deren Mischungen.

Im erfindungsgemäßen Verfahren können aliphatischer Carbonsäureester (I) und Amin (II) in beliebigen Verhältnissen miteinander zur Reaktion gebracht werden. Bevorzugt erfolgt die Umsetzung zwischen Ester und Amin mit molaren Verhältnissen von 10:1 bis 1:100, bevorzugt von 2:1 bis 1:10, speziell von 1,2:1 bis 1:3, jeweils bezogen auf die Molequivalente an Ester- und Amingruppen. In einer speziellen Ausführungsform werden Ester und Amin equimolar eingesetzt.

In vielen Fällen hat es sich als vorteilhaft erwiesen, mit einem Überschuss an Amin, das heißt molaren Verhältnissen von Amin zu Ester von mindestens 1,01:1,00 und insbesondere zwischen 50:1 und 1,02:1 wie beispielsweise zwischen 10:1 und 1,1:1 zu arbeiten. Dabei werden die Estergruppen praktisch quantitativ zum Amid umgesetzt. Besonders vorteilhaft ist dieses Verfahren, wenn das eingesetzte Amin leicht flüchtig ist. Leicht flüchtig heißt hier, dass das Amin einen Siedepunkt bei Normaldruck von vorzugsweise unterhalb 200 °C und besonders bevorzugt unterhalb 160 °C wie beispielsweise unterhalb 100 °C besitzt und sich somit destillativ vom Amid abtrennen lässt.

Für den Fall, dass der aliphatische Kohlenwasserstoffrest R³ eine oder mehrere Hydroxylgruppen trägt, erfolgt die Umsetzung zwischen Carbonsäureester (I) und Amin (II) bevorzugt mit molaren Verhältnissen von 100:1 bis 1:1, bevorzugt von 10:1 bis 1,001:1 und speziell von 5:1 bis 1,01:1 wie beispielsweise von 2:1 bis 1,1:1, jeweils bezogen auf die Molequivalente an Estergruppen und Aminogruppen im Reaktionsgemisch.

In einer bevorzugten Ausführungsform wird zur Beschleunigung bzw. zur Vervollständigung der Reaktion in Gegenwart von Katalysatoren gearbeitet. Vorzugsweise arbeitet man dabei in Gegenwart eines basischen Katalysators oder Gemischen aus mehreren dieser Katalysatoren. Als basische Katalysatoren werden im Rahmen der vorliegenden Erfindung ganz allgemein solche basischen Verbindungen eingesetzt, die geeignet sind, die Amidierung von Carbonsäureestern mit Aminen zu Carbonsäureamiden zu beschleunigen. Beispiele geeigneter Katalysatoren sind anorganische und organische Basen wie beispielsweise Metallhydroxide, -oxide, -carbonate oder -alkoxide. In einer bevorzugten Ausführungsform wird der basische Katalysator ausgewählt aus der Gruppe der Hydroxide, Oxide, Carbonate oder Alkoxide von Alkali- oder Erdalkalimetallen. Dabei sind Lithiumhydroxid, Natriumhydroxid, Kaliumhydroxid, Natriummethoxid, Kaliummethoxid, Natriumcarbonat, Natrium-tert.-butanolat, Kalium-tert.-butanolat und Kaliumcarbonat ganz besonders bevorzugt. Auch Cyanidionen sind als Katalysator geeignet. Diese Substanzen können in fester Form oder als Lösung wie beispielsweise als alkoholische Lösung eingesetzt werden. Die Menge der eingesetzten Katalysatoren hängt dabei von der Aktivität und Stabilität des Katalysators bei den gewählten Reaktionsbedingungen ab und ist der jeweiligen Reaktion anzupassen. Die Menge des einzusetzenden Katalysators kann dabei in weiten Grenzen variieren. Oftmals hat es sich bewährt, mit 0,1 bis 2,0 mol Base wie beispielsweise mit 0,2 bis 1,0 mol Base pro Mol eingesetztem Amin zu arbeiten. Besonders bevorzugt werden katalytische Mengen der oben genannten, reaktionsbeschleunigend wirkenden Verbindungen eingesetzt, bevorzugt im Bereich zwischen 0,001 und 10 Gew.-%, besonders bevorzugt im Bereich von 0,01 bis 5 Gew.-% wie beispielsweise zwischen 0,02 und 2 Gew.-%, bezogen auf die eingesetzte Menge an Carbonsäureester und Amin.

Die erfindungsgemäße Herstellung der Amide erfolgt durch Vermischen von Carbonsäureester und Amin sowie gegebenenfalls Katalysator und nachfolgende Bestrahlung des Reaktionsgemischs mit Mikrowellen in einem Reaktionsrohr, dessen Längsachse sich in der Ausbreitungsrichtung der Mikrowellen in einem Monomode-Mikrowellenapplikators befindet.

Die Bestrahlung des Reaktionsgemischs mit Mikrowellen erfolgt in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die im Hohlleiter nicht absorbierten Mikrowellen an seinem Ende reflektiert. Bevorzugt wird die Länge des Hohlraumresonators so dimensioniert, dass sich in ihm eine stehende Welle ausbildet. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen-Symmetrieachse ein Maximum und nimmt zur Mantelfläche hin auf den Wert null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 3 bis 50 von speziell 4 bis 20 wie beispielsweise drei, vier, fünf, sechs, sieben, acht, neun oder zehn.

Die E₀₁ₙ-Mode des Hohlraumresonators wird in Englischer Sprache auch als TM₀₁ₙ-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsgemischs mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Reaktionsrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgemischs mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgemischs mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgemischs mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis ca. 50 cm, insbesondere zwischen 2 mm und 35 cm und speziell zwischen 5 mm und 15 cm wie beispielsweise zwischen 10 mm und 7 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einem Wärmetauscher versehen. Damit sind Reaktionen in einem sehr weiten Druck- und Temperaturbereich möglich.

Die Herstellung des Reaktionsgemischs aus Ester, Amin und gegebenenfalls Katalysator und/oder Lösemittel kann kontinuierlich, diskontinuierlich oder auch in semi-Batch-Prozessen durchgeführt werden. So kann das Reaktionsgemisch in einem vorgelagerten (semi)-Batch Prozess hergestellt werden wie beispielsweise in einem Rührbehälter. In einer bevorzugten Ausführungsform werden die Edukte Amin und Carbonsäureester, beide unabhängig voneinander gegebenenfalls mit Lösemittel verdünnt, erst kurz vor dem Eintritt in das Reaktionsrohr vermischt. So hat es sich insbesondere bei Einsatz nicht unbegrenzt miteinander mischbarer Edukte bewährt, die Mischung von Amin und Ester in einer Mischstrecke vorzunehmen, aus der das Reaktionsgemisch in das Reaktionsrohr gefördert wird. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Reaktionsrohr zugesetzt werden. Bevorzugt werden Katalysatoren in flüssiger Form, zum Beispiel als Lösung in einem der Edukte oder in einem unter den Reaktionsbedingungen inerten Lösemittel eingesetzt. Auch heterogene Systeme können nach dem erfindungsgemäßen Verfahren umgesetzt werden, wobei entsprechende technische Vorrichtungen zum Fördern des Reaktionsgutes erforderlich sind.

Das Reaktionsgemisch kann in das Reaktionsrohr entweder an dem durch das Innenleiterrohr geführte Ende eingespeist werden als auch an dem entgegen gesetzten Ende. Das Reaktionsgemisch kann folglich parallel oder antiparallel zur Ausbreitungsrichtung der Mikrowellen durch den Mikrowellenapplikator geführt werden.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators sowie der eingestrahlten Mikrowellenleistung werden die Reaktionsbedingungen bevorzugt so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung, mehrfach das Reaktionsrohr durchlaufen. Vielfach hat sich bewährt, wenn das Reaktionsprodukt unmittelbar nach Verlassen des Reaktionsrohres z. B. durch Mantelkühlung oder Entspannung abgekühlt wird. Bei langsamer verlaufenden Reaktionen hat es sich oftmals bewährt, das Reaktionsprodukt nach Verlassen des Reaktionsrohres noch eine gewisse Zeit bei Reaktionstemperatur zu halten.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Besonders bewährt hat sich die Durchführung der Umsetzung bei Temperaturen zwischen 120 und maximal 400 °C, insbesondere zwischen 135 maximal 350 °C und speziell zwischen 155 und maximal 300 °C wie beispielsweise bei Temperaturen zwischen 180 und 270 °C.

Die Dauer der Mikrowellenbestrahlung hängt von verschiedenen Faktoren wie beispielsweise der Geometrie des Reaktionsrohres, der eingestrahlten Mikrowellenenergie, der speziellen Reaktion und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Hohlraumresonators die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt. In einer weiteren bevorzugten Ausführungsform wird der Katalysator, sofern anwesend, direkt nach Verlassen des Reaktionsrohres neutralisiert.

Bevorzugt wird die Umsetzung bei Drücken zwischen Atmosphärendruck und 500 bar, besonders bevorzugt zwischen 1,5 bar und 150 bar, insbesondere zwischen 3 bar und 100 bar und speziell zwischen 1,5 bar und 100 bar wie beispielsweise zwischen 10 bar und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder oberhalb des während der Reaktion gebildeten Alkohols gearbeitet wird. Besonders bevorzugt wird der Druck so hoch eingestellt, dass das Reaktionsgemisch während der Mikrowellenbestrahlung im flüssigen Zustand verbleibt und nicht siedet.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Es hat sich bewährt, in Gegenwart von Lösemitteln zu arbeiten um beispielsweise die Viskosität des Reaktionsmediums abzusenken und/oder das Reaktionsgemisch, sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen.

Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, Shellsol^{®} AB, Solvesso^{®} 150, Solvesso^{®} 200, Exxsol^{®}, Isopar^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

In einer weiteren bevorzugten Ausführungsform wird das erfindungsgemäße Verfahren in Lösemitteln mit höheren ε"-Werten von beispielsweise 5 und höher wie insbesondere mit ε"-Werten von 10 und höher durchgeführt. Diese Ausführungsform hat sich insbesondere bei der Umsetzung von Reaktionsgemischen bewährt, die selbst, das heißt ohne Anwesenheit von Löse- und/oder Verdünnungsmitteln nur eine sehr geringe Mikrowellenabsorption zeigen. So hat sich diese Ausführungsform insbesondere bei Reaktionsgemischen bewährt, die einen dielektrischen Verlust ε" von weniger als 10 und vorzugsweise weniger als 1 aufweisen. Die durch die Lösemittelzugabe oftmals beobachtete beschleunigte Aufheizung des Reaktionsgemischs erfordert jedoch Maßnahmen zur Einhaltung der Maximaltemperatur.

Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

In einer weiteren bevorzugten Ausführungsform werden dem Reaktionsgemisch in diesem unlösliche, stark Mikrowellen absorbierende Substanzen zugegeben. Diese führen zu einer starken lokalen Erhitzung des Reaktionsgemischs und in der Folge zu weiter beschleunigten Reaktionen. Ein geeigneter derartiger Wärmesammler ist beispielsweise Graphit.

Als Mikrowellen werden elektromagnetische Strahlen mit einer Wellenlänge zwischen etwa 1 cm und 1 m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche, medizinische, häusliche und ähnliche Anwendungen freigegebenen Frequenzen verwendet wie beispielsweise mit Frequenzen von 915 MHz, 2,45 GHz, 5,8 GHz oder 24, 12 GHz.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Reaktionsrohrs und damit des Reaktionsvolumens sowie der Dauer der erforderlichen Bestrahlung. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird die Reaktion in einem druckfesten, chemisch inerten Rohr durchgeführt, wobei gegebenenfalls die Edukte sowie Produkte und, sofern anwesend, Lösemittel zu einem Druckaufbau führen können. Nach Beendigung der Reaktion kann der Überdruck durch Entspannung zur Verflüchtigung und Abtrennung von leicht flüchtigen Komponenten sowie gegebenenfalls Lösemittel und/oder zur Abkühlung des Reaktionsprodukts verwendet werden. Der als Nebenprodukt gebildete Alkohol kann nach dem Abkühlen und/oder Entspannen durch übliche Verfahren wie beispielsweise Phasentrennung, Destillation, Strippen, Flashen und/oder Absorption abgetrennt werden. Oftmals kann der Alkohol auch im Produkt verbleiben.

Zur Erzielung besonders hoher Umsetzungsgrade hat es sich in vielen Fällen bewährt, das erhaltene Reaktionsprodukt gegebenenfalls nach Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Üblicherweise fallen über den erfindungsgemäßen Weg hergestellte Amide in einer für die weitere Verwendung ausreichenden Reinheit an, so dass keine weiteren Auf- oder Nacharbeitungsschritte erforderlich sind. Für spezielle Anforderungen können sie jedoch nach üblichen Reinigungsverfahren wie beispielsweise Destillation, Umkristallisation, Filtration bzw. chromatographische Verfahren weiter aufgereinigt werden.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von beispielsweise mehr als 10 kW oder mehr als 100 kW zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es überraschend, dass trotz der im kontinuierlich durchflossenen Strömungsrohr nur sehr kurzen Verweildauer des Reaktionsguts im Mikrowellenfeld eine sehr weitgehende Amidierung mit Umsätzen im Allgemeinen von über 80 %, oftmals auch über 90 % wie beispielsweise über 95 % bezogen auf die im Unterschuss eingesetzte Komponente stattfindet, ohne Bildung nennenswerter Mengen an Nebenprodukten. Weiterhin überraschend war, dass sich die genannten hohen Umsätze unter diesen Reaktionsbedingungen ohne Abtrennung des bei der Aminolyse gebildeten Alkohols erzielen lassen. Bei einer entsprechenden Umsetzung dieser Reaktionsgemische in einem Strömungsrohr gleicher Dimensionierung unter thermischer Mantelheizung werden zur Erzielung geeigneter Reaktionstemperaturen extrem hohe Wandtemperaturen benötigt, die zur Bildung gefärbter Spezies führten, aber bei gleichem Zeitintervall nur geringfügige Amidbildung bewirken.

Das erfindungsgemäße Verfahren erlaubt somit eine sehr schnelle, energiesparende und kostengünstige Herstellung von Carbonsäureamiden in hohen Ausbeuten und mit hoher Reinheit in großtechnischen Mengen. Bei diesem Verfahren fallen - neben dem Alkohol - keine wesentlichen Mengen an Nebenprodukten an. Derartig schnelle und selektive Umsetzungen sind nach klassischen Methoden nicht zu erzielen und waren alleine durch Heizen auf hohe Temperaturen nicht zu erwarten.

### Beispiele

Die Umsetzungen der Reaktionsgemische unter Mikrowellenbestrahlung erfolgten in einem Keramikrohr (60 x 1 cm), das sich axialsymmetrisch in einem zylindrischen Hohlraumresonator (60 x 10 cm) befand. An einer der Stirnseiten des Hohlraumresonators verlief das Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode), in dem sich eine stehende Welle ausbildete.

Die Mikrowellenleistung wurde über die Versuchsdauer jeweils in der Art eingestellt, dass die gewünschte Temperatur des Reaktionsguts am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Reaktionszone (etwa 15 cm Strecke in einer isolierten Edelstahlkapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurückgespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet.

Mittels einer Hochdruckpumpe und eines geeigneten Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die Ester und Amin enthaltenden Reaktionsgemische wurden mit einer konstanten Flussrate durch das Reaktionsrohr gepumpt und die Verweilzeit in der Bestrahlungszone durch Modifizierung der Strömungsgeschwindigkeit eingestellt.

Die Analytik der Produkte erfolgte mittels ¹H-NMR-Spektroskopie bei 500 MHz in CDCl₃.

### Beispiel 1: Herstellung von Cocosfettsäure-(N',N'-dimethylaminopropyl)amid

In einem 10 l Büchi-Rührautoklaven mit Gaseinleitungsrohr, Rührer, Innenthermometer und Druckausgleich wurden 4,6 kg Cocosfett (6 mol / Molekulargwicht 764 g/mol) vorgelegt und auf 55 °C erwärmt. Bei dieser Temperatur wurden langsam 2,76 kg N,N-Dimethylaminopropylamin (27 mol) sowie 100 g Natriummethylat als Katalysator zugegeben und unter Rühren homogenisiert.

Das so erhaltene Reaktionsgemisch wurde bei einem Arbeitsdruck von 35 bar kontinuierlich mit 5 l/h durch das Reaktionsrohr gepumpt und einer Mikrowellenleistung von 2,5 kW ausgesetzt, von denen 92 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 271 °C. Das Reaktionsgemisch wurde direkt nach Verlassen des Reaktors auf Raumtemperatur abgekühlt.

Das Reaktionsprodukt war leicht gelblich gefärbt. Nach Abtrennung von entstandenem Glycerin und überschüssigem N,N-Dimethylaminopropylamin wurden 5,4 kg Cocosfettsäure-(N',N'-dimethylaminopropyl)amid mit einer Reinheit von 95 % erhalten.

### Beispiel 2: Herstellung von N,N-Diethylcocosfettsäureamid

In einem 10 l Büchi-Rührautoklaven mit Gaseinleitungsrohr, Rührer, Innenthermometer und Druckausgleich wurden 4,2 kg Cocosfett (5,5 mol / Molekulargwicht 764 g/mol) vorgelegt und auf 45 °C erwärmt. Bei dieser Temperatur wurden langsam 2 kg Diethylamin (27 mol) sowie 100 g Natriumethylat als Katalysator zugegeben und unter Rühren homogenisiert.

Das so erhaltene Reaktionsgemisch wurde bei einem Arbeitsdruck von 35 bar kontinuierlich mit 4,5 l/h durch das Reaktionsrohr gepumpt und einer Mikrowellenleistung von 2,2 kW ausgesetzt, von denen 91 % vom Reaktionsgut absorbiert wurden. Die Verweilzeit des Reaktionsgemischs in der Bestrahlungszone betrug ca. 38 Sekunden. Am Ende des Reaktionsrohres hatte das Reaktionsgemisch eine Temperatur von 265 °C. Das Reaktionsgemisch wurde direkt nach Verlassen des Reaktors auf Raumtemperatur abgekühlt.

Das Reaktionsprodukt war leicht gelblich gefärbt. Nach Abtrennung von entstandenem Glycerin und überschüssigem Diethylamin wurden 3,7 kg N,N-Diethylcocosfettsäureamid mit einer Reinheit von 97 % erhalten.

### Beispiel 3: Herstellung von N,N-Dimethylacetamid

In einem 10 l Büchi-Rührautoklaven mit Gaseinleitungsrohr, Rührer, Innenthermometer und Druckausgleich wurden 1,76 kg Essigsäureethylester (20 mol) vorgelegt und langsam mit 6 kg Diethylamin (40 mol als 30 %ige Lösung in Ethanol) sowie 100 g Natriumethylat als Katalysator zugegeben und unter Rühren homogenisiert.

Das so erhaltene Reaktionsgemisch wurde bei einem Arbeitsdruck von 35 bar kontinuierlich mit 6 l/h durch das Reaktionsrohr gepumpt und einer Mikrowellenleistung von 3,2 kW ausgesetzt, von denen 95 % vom Reaktionsgut absorbiert wurden. Die Verweilzeit des Reaktionsgemischs in der Bestrahlungszone betrug ca. 28 Sekunden. Am Ende des Reaktionsrohres hatte das Reaktionsgemisch eine Temperatur von 278 °C. Das Reaktionsgemisch wurde direkt nach Verlassen des Reaktors auf Raumtemperatur abgekühlt.

Es wurde ein Umsatz von 88 % d. Th. erreicht. Das Reaktionsprodukt war farblos. Nach destillativer Auftrennung des Rohproduktes wurden 1,5 kg N,N-Dimethylacetamid mit einer Reinheit von >99 % erhalten.

## Patentansprüche

1. Kontinuierllches Verfahren zur Verstellung von Amiden aliphatischer Carbonsäuren, in dem mindestens ein Carbonsäureester der Formel (I)
R³-COOR⁴ (I)
worin
R³ für Wasserstoff oder einen gegebenenfalls substituierten aliphatischen Kohlenwasserstoffrest mit 1 bis 100 Kohlenstoffatomen und
R⁴ für einen gegebenenfalls substituierten Kohlenwasserstoffrest mit 1 bis 30 Kohlenstoffatomen steht,
oder worin R³ und R⁴ einen gegebenenfalls substituierten Ring mit 5, 6 oder 7 Ringgliedern bilden
mit mindestens einem Amin der Formel (II)
HNR¹R² (II)
worin R¹ und R² unabhängig voneinander für Wasserstoff oder einen gegebenenfalls substituierten Kohlenwasserstoffrest mit 1 bis 100 C-Atomen stehen,
und worin R¹ und R² unabhängig voneinander eine gegebenenfalls substituierte heteroaromatische Gruppe mit 5 bis 12 Ringgliedern sein können,
und worin R¹ und R² unabhängig voneinander für einen mit Heteroatomen unterbrochenen Alkylrest stehen können,
und worin R¹ und R² zusammen mit dem Stickstoffatom, an das sie gebunden sind, einen Ring bilden können,
unter Mikrowellenbestrahlung des Reaktionsgemischs in einem weitgehend mikrowellentransparenten Reaktionsrohr innerhalb eines mit einem Mikrowellengenerator über Wellenleiter verbundenen Hohlleiters" dessen Längsachse sich in der Ausbreitungsrichtung der Mikrowellen eines Monomode-Mikrowellenapplikators befindet, zum Carbonsäureamid umgesetzt wird.

2. Verfahren nach Anspruch 1, bei dem der Mikrowellenapplikator als Hohlraumresonator ausgestaltet ist.

3. Verfahren nach Anspruch 1 und/oder 2, bei dem der Mikrowellenapplikator als Hohlraumresonator vom Reflexionstyp ausgestaltet ist.

4. Verfahren nach einem oder mehreren der Ansprüche 1 bis 3, bei dem das Reaktionsrohr axial mit einer zentralen Symmetrieachse des Hohlleiters fluchtet.

5. Verfahren nach einem oder mehreren der Ansprüche 1 bis 4, bei dem die Bestrahlung des Reaktionsgemisches in einem Hohlraumresonator mit koaxialem Übergang der Mikrowellen erfolgt.

6. Verfahren nach einem oder mehreren der Ansprüche 1 bis 5, bei dem der Hohlraumresonator im E₀₁ₙ-Mode betrieben wird, wobei n eine ganze Zahl von 1 bis 200 ist.

7. Verfahren nach einem oder mehreren der Ansprüche 1 bis 6, bei dem sich im Hohlraumresonator eine stehende Welle ausbildet.

8. Verfahren nach einem oder mehreren der Ansprüche 1 bis 7, bei dem das Reaktionsgut durch die Mikrowellenbestrahlung auf Temperaturen zwischen 120 und 500 °C erhitzt wird.

9. Verfahren nach einem oder mehreren der Ansprüche 1 bis 8, bei dem die Mikrowellenbestrahlung bei Drücken oberhalb des Atmosphärendrucks erfolgt.

10. Verfahren nach einem oder mehreren der Ansprüche 1 bis 9, bei dem R³ 2 bis 26 C-Atome umfasst.

11. Verfahren nach einem oder mehreren der Ansprüche 1 bis 10, bei dem R³ mindestens eine weitere Estergruppe -COOR⁴ trägt, worin R⁴ für einen gegebenenfalls substituierten Kohlenwasserstoffrest mit 1 bis 30 Kohlenstoffatomen steht.

12. Verfahren nach einem oder mehreren der Ansprüche 1 bis 11, bei dem R³ für einen gegebenenfalls substituierten aliphatischen Kohlenwasserstoffrest mit 2-100 C-Atomen steht, der mindestens eine C=C-Doppelbindung enthält.

13. Verfahren nach einem oder mehreren der Ansprüche 1 bis 12, bei dem R⁴ 2 bis 24 C-Atome umfasst.

14. Verfahren nach einem oder mehreren der Ansprüche 1 bis 13, bei dem R⁴ eine oder mehrere weitere Hydroxylgruppen trägt.

15. Verfahren nach einem oder mehreren der Ansprüche 1 bis 14, bei dem die Verbindung der Formel (I) ein Ester einer aliphatischen Carbonsäure mit einem Monoalkohol mit 1 bis 4 C-Atomen ist.

16. Verfahren nach einem oder mehreren der Ansprüche 1 bis 14, bei dem die Verbindung der Formel (I) ein Ester gleicher oder verschiedener, gegebenenfalls substituierter aliphatischer Carbonsäuren mit einem Polyalkohol mit 2 bis 6 Hydroxylgruppen ist.

17. Verfahren nach einem oder mehreren der Ansprüche 1 bis 16, bei dem die Verbindung der Formel (I) ein intramolekularer Ester ist.

18. Verfahren nach einem oder mehreren der Ansprüche 1 bis 17, bei dem R¹ und/oder R² unabhängig voneinander aliphatische Reste mit 2 bis 24 C-Atomen sind.

19. Verfahren nach einem oder mehreren der Ansprüche 1 bis 17, bei dem R¹ und R² zusammen mit dem Stickstoffatom, an das sie gebunden sind, einen Ring mit 4 oder mehr Ringgliedern bilden.

20. Verfahren nach einem oder mehreren der Ansprüche 1 bis 17, bei dem R¹ und/oder R² unabhängig voneinander für eine gegebenenfalls substituierte, C₆-C₁₂-Arylgruppe oder eine gegebenenfalls substituierte heteroaromatische Gruppe mit 5 bis 12 Ringgliedern stehen.

21. Verfahren nach einem oder mehreren der Ansprüche 1 bis 17, bei dem R¹ und/oder R² unabhängig voneinander für Reste der Formel (V)
-(R⁷O)ₙ-R⁸ (V)
stehen, worin
R⁷ für eine Alkylengruppe mit 2 bis 6 C-Atomen oder Mischungen daraus,
R⁸ für Wasserstoff, einen Kohlenwasserstoffrest mit 1 bis 24 C-Atomen oder eine Gruppe der Formel -R⁷-NR¹¹R¹²,
n für eine Zahl zwischen 2 und 50, und
R¹¹, R¹² unabhängig voneinander für einen aliphatischen Rest mit 1 bis 24 C-Atomen, eine Aryl- oder Heteroarylgruppe mit 5 bis 12 Ringgliedern, eine Poly(oxyalkylen)gruppe mit 1 bis 50 Poly(oxyalkylen)einheiten, wobei sich die Poly(oxyalkylen)einheiten von Alkylenoxideinheiten mit 2 bis 6 C-Atomen ableiten, oder R¹¹ und R¹² gemeinsam mit dem Stickstoffatom, an das sie gebunden sind, einen Ring mit 4, 5, 6 oder mehr Ringgliedern bilden, stehen.

22. Verfahren nach einem oder mehreren der Ansprüche 1 bis 17, bei dem R¹ und/oder R² unabhängig voneinander für Reste der Formel (VI)
-[R⁹-N(R¹⁰)]ₘ-(R¹⁰) (VI)
stehen, worin
R⁹ für eine Alkylengruppe mit 2 bis 6 C-Atomen oder Mischungen daraus steht,
jedes R¹⁰ unabhängig voneinander für Wasserstoff, einen Alkyl- oder Hydroxyalkylrest mit bis zu 24 C-Atomen, einen Polyoxyalkylenrest -(R⁷-O)ₚ-R⁸, oder einen Polyiminoalkylenrest -[R⁹-N(R¹⁰)]_{q}-(R¹⁰) stehen,
R⁷ für eine Alkylengruppe mit 2 bis 6 C-Atomen oder Mischungen daraus,
R⁸ für Wasserstoff, einen Kohlenwasserstoffrest mit 1 bis 24 C-Atomen oder eine Gruppe der Formel -R⁷-NR¹¹R¹²,
R¹¹, R¹² unabhängig voneinander für einen aliphatischen Rest mit 1 bis 24 C-Atomen, eine Aryl- oder Heteroarylgruppe mit 5 bis 12 Ringgliedern, eine Poly(oxyalkylen)gruppe mit 1 bis 50 Poly(oxyalkylen)einheiten, wobei sich die Poly(oxyalkylen)einheiten von Alkylenoxideinheiten mit 2 bis 6 C-Atomen ableiten, oder R¹¹ und R¹² gemeinsam mit dem Stickstoffatom, an das sie gebunden sind, einen Ring mit 4, 5, 6 oder mehr Ringgliedern bilden, und q und p unabhängig voneinander für 1 bis 50 stehen und
m für eine Zahl von 1 bis 20 steht.

23. Verfahren nach einem oder mehreren der Ansprüche 1 bis 18 und/oder 21 bis 25, bei dem das Amin der Formel (II) ein primäres Amin ist.

24. Verfahren nach einem oder mehreren der Ansprüche 1 bis 22, bei dem das Amin der Formel (II) ein sekundäres Amin ist.

## Claims

1. A continuous process for preparing amides of aliphatic carboxylic acids, in which at least one carboxylic ester of the formula (I)
R³-COOR⁴ (I)
in which
R³ is hydrogen or an optionally substituted aliphatic hydrocarbyl radical having 1 to 100 carbon atoms and
R⁴ is an optionally substituted hydrocarbyl radical having 1 to 30 carbon atoms, or in which R³ and R⁴ form an optionally substituted ring having 5, 6 or 7 ring members
is reacted with at least one amine of the formula (II)
HNR¹R² (II)
in which R¹ and R² are each independently hydrogen or an optionally substituted hydrocarbyl radical having 1 to 100 carbon atoms,
and in which R¹ and R² are each independently an optionally substituted heteroaromatic group having 5 to 12 ring members,
and in which R¹ and R² are each independently an alkyl radical interrupted by heteroatoms,
and in which R¹ and R² together with the nitrogen atom to which they are bonded may form a ring,
under microwave irradiation of the reaction mixture in a substantially microwave-transparent reaction tube within a hollow conductor which is connected via waveguides to a microwave generator and whose longitudinal axis is in the direction of propagation of the microwaves of a monomode microwave applicator to give the carboxamide.

2. The process as claimed in claim 1, in which the microwave applicator is configured as a cavity resonator.

3. The process as claimed in claim 1 and/or 2, in which the microwave applicator is configured as a cavity resonator of the reflection type.

4. The process as claimed in one or more of claims 1 to 3, in which the reaction tube is aligned axially with a central axis of symmetry of the hollow conductor.

5. The process as claimed in one or more of claims 1 to 4, in which the reaction mixture is irradiated in a cavity resonator with a coaxial transition of the microwaves.

6. The process as claimed in one or more of claims 1 to 5, in which the cavity resonator is operated in E₀₁ₙ mode where n is an integer from 1 to 200.

7. The process as claimed in one or more of claims 1 to 6, in which a standing wave forms in the cavity resonator.

8. The process as claimed in one or more of claims 1 to 7, in which the reaction mixture is heated by the microwave irradiation to temperatures between 120 and 500°C.

9. The process as claimed in one or more of claims 1 to 8, in which the microwave irradiation is effected at pressures above atmospheric pressure.

10. The process as claimed in one or more of claims 1 to 9, in which R³ comprises 2 to 26 carbon atoms.

11. The process as claimed in one or more of claims 1 to 10, in which R³ bears at least one further ester group -COOR⁴ in which R⁴ is an optionally substituted hydrocarbyl radical having 1 to 30 carbon atoms.

12. The process as claimed in one or more of claims 1 to 11, in which R³ is an optionally substituted aliphatic hydrocarbyl radical which has 2-100 carbon atoms and contains at least one C=C double bond.

13. The process as claimed in one or more of claims 1 to 12, in which R⁴ comprises 2 to 24 carbon atoms.

14. The process as claimed in one or more of claims 1 to 13, in which R⁴ bears one or more further hydroxyl groups.

15. The process as claimed in one or more of claims 1 to 14, in which the compound of the formula (I) is an ester of an aliphatic carboxylic acid with a monoalcohol having 1 to 4 carbon atoms.

16. The process as claimed in one or more of claims 1 to 14, in which the compound of the formula (I) is an ester of identical or different, optionally substituted carboxylic acids with a polyalcohol having 2 to 6 hydroxyl groups.

17. The process as claimed in one or more of claims 1 to 16, in which the compound of the formula (I) is an intramolecular ester.

18. The process as claimed in one or more of claims 1 to 17, in which R¹ and/or R² are each independently aliphatic radicals having 2 to 24 carbon atoms.

19. The process as claimed in one or more of claims 1 to 17, in which R¹ and R² together with the nitrogen atom to which they are bonded form a ring having 4 or more ring members.

20. The process as claimed in one or more of claims 1 to 17, in which R¹ and/or R² are each independently an optionally substituted C₆-C₁₂-aryl group or an optionally substituted heteroaromatic group having 5 to 12 ring members.

21. The process as claimed in one or more of claims 1 to 17, in which R¹ and/or R² are each independently radicals of the formula (V)
-(R⁷O)ₙ-R⁸ (V)
in which
R⁷ is an alkylene group having 2 to 6 carbon atoms or mixtures thereof,
R⁸ is hydrogen, a hydrocarbyl radical having 1 to 24 carbon atoms or a group of the formula -R⁷-NR¹¹R¹²,
n is a number from 2 to 50, and
R¹¹, R¹² are each independently an aliphatic radical having 1 to 24 carbon atoms, an aryl or heteroaryl group having 5 to 12 ring members, a poly-(oxyalkylene) group having 1 to 50 poly(oxyalkylene) units, where the poly(oxyalkylene) units derive from alkylene oxide units having 2 to 6 carbon atoms, or R¹¹ and R¹² together with the nitrogen atom to which they are bonded form a ring having 4, 5, 6 or more ring members.

22. The process as claimed in one or more of claims 1 to 17, in which R¹ and/or R² are each independently radicals of the formula (VI)
-[R⁹-N(R¹⁰)]ₘ-(R¹⁰) (VI)
in which
R⁹ is an alkylene group having 2 to 6 carbon atoms or mixtures thereof,
each R¹⁰ is independently hydrogen, an alkyl or hydroxyalkyl radical having up to 24 carbon atoms, a polyoxyalkylene radical -(R⁷-O)ₚ-R⁸ or a polyiminoalkylene radical -[R⁹-N(R¹⁰)]_{q}-(R¹⁰),
R⁷ is an alkylene group having 2 to 6 carbon atoms or mixtures thereof,
R⁸ is hydrogen, a hydrocarbyl radical having 1 to 24 carbon atoms or a group of the formula -R⁷-NR¹¹R¹²,
R¹¹, R¹² are each independently an aliphatic radical having 1 to 24 carbon atoms, an aryl or heteroaryl group having 5 to 12 ring members, a poly(oxyalkylene) group having 1 to 50 poly(oxyalkylene) units, where the poly(oxyalkylene) units derive from alkylene oxide units having 2 to 6 carbon atoms, or R¹¹ and R¹² together with the nitrogen atom to which they are bonded form a ring having 4, 5, 6 or more ring members, and q and p are each independently from 1 to 50 and
m is a number from 1 to 20.

23. The process as claimed in one or more of claims 1 to 18 and/or 21 to 25, in which the amine of the formula (II) is a primary amine.

24. The process as claimed in one or more of claims 1 to 22, in which the amine of the formula (II) is a secondary amine.

## Revendications

1. Procédé continu pour la préparation d'amides d'acides carboxyliques aliphatiques, dans lequel on transforme au moins un ester d'acide carboxylique de formule (I)
R³-COOR⁴ (I)
dans laquelle
R³ représente hydrogène ou un radical hydrocarboné aliphatique le cas échéant substitué comprenant 1 à 100 atomes de carbone et
R⁴ représente un radical hydrocarboné le cas échéant substitué comprenant 1 à 30 atomes de carbone,
ou dans laquelle R³ et R⁴ représentent un cycle le cas échéant substitué de 5, 6 ou 7 chaînons
avec au moins une amine de formule (II)
HNR¹R² (II)
dans laquelle R¹ et R² représentent, indépendamment l'un de l'autre, hydrogène ou un radical hydrocarboné le cas échéant substitué comprenant 1 à 100 atomes de carbone,
et dans laquelle R¹ et R² peuvent représenter indépendamment l'un de l'autre un groupe hétéroaromatique le cas échéant substitué de 5 à 12 chaînons,
et dans laquelle R¹ et R² peuvent représenter, indépendamment l'un de l'autre, un radical alkyle interrompu par des hétéroatomes,
et dans laquelle R¹ et R² peuvent former, ensemble avec l'atome d'azote auquel ils sont liés, un cycle,
sous une irradiation par des micro-ondes du mélange réactionnel dans un tube de réaction dans une large mesure transparent aux micro-ondes dans un conducteur creux relié via des guides d'ondes à un générateur de micro-ondes, dont l'axe longitudinal se trouve dans le sens de propagation des micro-ondes d'un dispositif d'application de micro-ondes monomodales, en amide d'acide carboxylique.

2. Procédé selon la revendication 1, dans lequel le dispositif d'application de micro-ondes est réalisé sous forme de cavité creuse.

3. Procédé selon la revendication 1 et/ou 2, dans lequel le dispositif d'application de micro-ondes est réalisé sous forme d'une cavité résonante de type réflexion.

4. Procédé selon l'une ou plusieurs des revendications 1 à 3, dans lequel le tube de réaction est axialement aligné sur un axe de symétrie central du conducteur creux.

5. Procédé selon l'une ou plusieurs des revendications 1 à 4, dans lequel l'irradiation du mélange réactionnel a lieu dans une cavité résonante avec une transition coaxiale des micro-ondes.

6. Procédé selon l'une ou plusieurs des revendications 1 à 5, dans lequel la cavité résonante est exploitée dans un mode E₀₁ₙ, n représentant un nombre entier de 1 à 200.

7. Procédé selon l'une ou plusieurs des revendications 1 à 6, dans lequel une onde stationnaire se forme dans la cavité résonante.

8. Procédé selon l'une ou plusieurs des revendications 1 à 7, dans lequel le produit de réaction est chauffé à des températures entre 120 et 500°C par l'irradiation par des micro-ondes.

9. Procédé selon l'une ou plusieurs des revendications 1 à 8, dans lequel l'irradiation par des micro-ondes a lieu à des pressions supérieures à la pression atmosphérique.

10. Procédé selon l'une ou plusieurs des revendications 1 à 9, dans lequel R³ comprend 2 à 26 atomes de carbone.

11. Procédé selon l'une ou plusieurs des revendications 1 à 10, dans lequel R³ porte au moins un autre groupe ester -COOR⁴, dans lequel R⁴ représente un radical hydrocarboné le cas échéant substitué comprenant 1 à 30 atomes de carbone.

12. Procédé selon l'une ou plusieurs des revendications 1 à 11, dans lequel R³ représente un radical hydrocarboné aliphatique le cas échéant substitué comprenant 2 à 100 atomes de carbone, qui comprend au moins une double liaison C=C.

13. Procédé selon l'une ou plusieurs des revendications 1 à 12, dans lequel R⁴ comprend 2 à 24 atomes de carbone.

14. Procédé selon l'une ou plusieurs des revendications 1 à 13, dans lequel R⁴ porte un ou plusieurs groupes hydroxyle.

15. Procédé selon l'une ou plusieurs des revendications 1 à 14, dans lequel le composé de formule (I) représente un ester d'un acide carboxylique aliphatique avec un monoalcool comprenant 1 à 4 atomes de carbone.

16. Procédé selon l'une ou plusieurs des revendications 1 à 14, dans lequel le composé de formule (I) représente un ester d'acides carboxyliques aliphatiques identiques ou différents, le cas échéant substitués, avec un polyalcool comprenant 2 à 6 groupes hydroxyle.

17. Procédé selon l'une ou plusieurs des revendications 1 à 16, dans lequel le composé de formule (I) est un ester intramoléculaire.

18. Procédé selon l'une ou plusieurs des revendications 1 à 17, dans lequel R¹ et/ou R² représentent, indépendamment l'un de l'autre, des radicaux aliphatiques comprenant 2 à 24 atomes de carbone.

19. Procédé selon l'une ou plusieurs des revendications 1 à 17, dans lequel R¹ et R² forment ensemble avec l'atome d'azote auquel ils sont liés un cycle de 4 chaînons ou plus.

20. Procédé selon l'une ou plusieurs des revendications 1 à 17, dans lequel R¹ et/ou R² représentent, indépendamment l'un de l'autre, un groupe C₆-C₁₂-aryle le cas échéant substitué ou un groupe hétéroaromatique le cas échéant substitué de 5 à 12 chaînons.

21. Procédé selon l'une ou plusieurs des revendications 1 à 17, dans lequel R¹ et/ou R² représentent, indépendamment l'un de l'autre, des radicaux de formule (V)
-(R⁷O)ₙ-R⁸ (V)
dans laquelle
R⁷ représente un groupe alkylène comprenant 2 à 6 atomes de carbone ou des mélanges de ceux-ci,
R⁸ représente hydrogène, un radical hydrocarboné comprenant 1 à 24 atomes de carbone ou un groupe de formule -R⁷-NR¹¹R¹²
n vaut un nombre entre 2 et 50, et
R¹¹, R¹² représentent, indépendamment l'un de l'autre, un radical aliphatique comprenant 1 à 24 atomes de carbone, un groupe aryle ou hétéroaryle de 5 à 12 chaînons, un groupe poly(oxyalkylène) comprenant 1 à 50 unités poly(oxyalkylène), les unités poly(oxyalkylène) étant dérivées d'unités d'oxyde d'alkylène comprenant 2 à 6 atomes de carbone, ou R¹¹ et R¹² représentent, ensemble avec l'atome d'azote, auquel ils sont liés, un cycle de 4, 5, 6 chaînons ou plus.

22. Procédé selon l'une ou plusieurs des revendications 1 à 17, dans lequel R¹ et/ou R² représentent, indépendamment l'un de l'autre, des radicaux de formule (VI)
-[R⁹N(R¹⁰)]ₘ-(R¹⁰) (VI)
dans laquelle
R⁹ représente un groupe alkylène comprenant 2 à 6 atomes de carbone ou des mélanges de ceux-ci,
chaque R¹⁰ représente, indépendamment l'un de l'autre, hydrogène, un radical alkyle ou hydroxyalkyle comprenant jusqu'à 24 atomes de carbone, un radical polyoxyalkylène -(R⁷-O)ₚ-R⁸, ou un radical polyiminoalkylène -[R⁹-N(R¹⁰)]_{q}-(R¹⁰),
R⁷ représente un groupe alkylène comprenant 2 à 6 atomes de carbone ou des mélanges de ceux-ci,
R⁸ représente hydrogène, un radical hydrocarboné comprenant 1 à 24 atomes de carbone ou un groupe de formule -R⁷-NR¹¹R¹²
R¹¹, R¹² représentent, indépendamment l'un de l'autre un radical aliphatique comprenant 1 à 24 atomes de carbone, un groupe aryle ou hétéroaryle de 5 à 12 chaînons, un groupe poly(oxyalkylène) comprenant 1 à 50 unités poly(oxyalkylène), les unités poly(oxyalkylène) étant dérivées d'unités d'oxyde d'alkylène comprenant 2 à 6 atomes de carbone, ou R¹¹ et R¹² représentent, ensemble avec l'atome d'azote, auquel ils sont liés, un cycle de 4, 5, 6 chaînons ou plus et q et p représentent, indépendamment l'un de l'autre, 1 à 20 et
m vaut un nombre de 1 à 20.

23. Procédé selon l'une ou plusieurs des revendications 1 à 18 et/ou 21 à 25, dans lequel l'amine de formule (II) est une amine primaire.

24. Procédé selon l'une ou plusieurs des revendications 1 à 22, dans lequel l'amine de formule (II) est une amine secondaire.
